# EUROPÄISCHE PATENTSCHRIFT

(11) **EP 0 537 187 B1**
(45) Veröffentlichungstag und Bekanntmachung des Hinweises auf die Patenterteilung: **14.09.1994**
(21) Anmeldenummer: 91910657.5
(22) Anmeldetag: 12.06.1991
(51) Int. Cl.: C07H 5/02, C07H 13/04, C07H 13/08

(54) **VERFAHREN ZUR HERSTELLUNG ACYLIERTER GLYCOSYLFLUORIDE**
PROCESS FOR PRODUCING ACYLATED GLYCOSYL FLUORIDES
PROCEDE DE PRODUCTION DE FLUORURES DE GLYCOSYL ACYLES

(30) Priorität: 02.07.1990 DE 4021001
(43) Veröffentlichungstag der Anmeldung: 21.04.1993
(73) Patentinhaber: HOECHST AKTIENGESELLSCHAFT, 65926 Frankfurt am Main (DE)
(72) Erfinder: WIESNER, Matthias, D-6500 Mainz (DE)
(86) Internationale Anmeldenummer: EP9101095
(87) Internationale Veröffentlichungsnummer: WO9200308

(56) Entgegenhaltungen:
- Acta Chemica Scandinavica, Vol. 16, No. 8, 1962, C. Pedersen: "Isomerisation of penta-0-acetyl-beta-D-glucopyranose with hydrogen fluoridw", pages 1831-1836, see the whole article (cited in the application)
- Zeitschrift für Chemie, Vol. 29, No. 12, Dexember 1989, R. Miethchen: Reaktionsverhalten der Kohlenhydrate in wasserfreiem, flüssigem Fluorwasserstoff", pages 425-434, see the whole article"

## Beschreibung

Die vorliegende Erfindung betrifft ein Verfahren zur Herstellung acylierter Glycosylfluoride durch Fluorierung von O-peracylierten oder partiell O-acylierten Sacchariden in wasserfreiem Fluorwasserstoff unter Zusatz des entsprechenden Carbonsäureanhydrids.

Am Sauerstoff veresterte, d.h. acylierte Glycosylfluoride sind außerordentlich nützliche Glycosylierungsreagenzien in der Kohlenhydrat-Chemie. Sie lassen sich unter Lewissäure-Katalyse in einfacher Weise mit Nucleophilen umsetzen.

Es ist bekannt, daß man acylierte Glycosylfluoride herstellen kann, indem man O-peracylierte Saccharide mit Fluorwasserstoff oder Pyridin-Polyhydrofluorid, oder Derivate mit freier anomerer Hydroxylgruppe mit Fluorierungsmitteln wie Diäthylaminoschwefeltrifluorid (DAST), reagieren läßt. Zusammenfassend geht dies aus den Arbeiten von F. Micheel und A. Klemer (Adv. Carbohydr. Chem. 16, 85 (1961), A. A. E. Penglis (Adv. Carbohydr. Chem. Biochem. 38, 195 (1981)) oder P. J. Card (J. Carbohydr. Chem. 4, 451 (1985)) hervor. Danach entstehen die gewünschten Produkte in Ausbeuten zwischen etwa 50 und 85 %, wobei die Reaktionszeiten bis zu 10 Stunden betragen können. DAST kommt wegen seines außerordentlich hohen Preises für technische Anwendungen kaum in Frage. Ausbeutemindernd wirken vor allem Umlagerungs- und Zersetzungsreaktionen, wie sie z.B. von C. Pedersen eingehend untersucht worden sind (Acta Chem. Scand. 17, 673 (1963)).

Bekannt ist weiterhin, daß ein Zusatz von Essigsäureanhydrid bei der Herstellung acetylierter Glycosylfluoride in wasserfreiem Fluorwasserstoff bei längeren Reaktionszeiten zu verstärkten Nebenreaktionen führt und die Ausbeute deutlich mindert (C. Pedersen, Acta Chem. Scand. 16, 1831 (1962)).

Überraschend wurde nun gefunden, daß die Fluorierung acylierter Kohlenhydratderivate in wasserfreiem Fluorwasserstoff durch einen Zusatz des entsprechenden Säureanhydrids bei kurzen Reaktionszeiten, d.h. unter 1 h, zu wesentlich weniger Nebenreaktionen und damit zu außerordentlich reinen Produkten in hoher Ausbeute führt.

Gegenstand der Erfindung ist nun ein Verfahren zur Herstellung von acylierten Glycosylfluoriden durch Fluorierung in wasserfreiem Fluorwasserstoff, das dadurch gekennzeichnet ist, daß dem Fluorwasserstoff vor der Reaktion ein Zusatz des entsprechenden Carbonsäureanhydrids zugefügt wird und die Reaktionslösung nach Reaktionszeiten von etwa 30 sec bis 60 min aufgearbeitet wird.

Durch die Erfindung werden gleich mehrere Vorteile gegenüber den herkömmlichen Verfahrensweisen erreicht: Umlagerungs- und Zersetzungsreaktionen, wie bei C. Pedersen (l.c.) beschrieben, treten offensichtlich erst sehr viel später auf. Zudem kann die Reaktion in sehr konzentrierten Lösungen durchgeführt werden, was den Verbrauch an Reagenzien auf ein Minimum reduziert. Da die Umsetzungen in der Regel fast augenblicklich erfolgen, genügen sehr kurze Reaktionszeiten, und es können nach Entfernung des Fluorwasserstoffs und der Carbonsäure, die bei wäßriger Aufarbeitung aus dem zugefügten Anhydrid entsteht, die reinen Glycosylfluoride isoliert werden.

Als peracylierte oder partiell acylierte Ausgangsverbindungen eignen sich von Monosacchariden wie Glucose, Galactose, Mannose, Gulose, Talose u.a. einschließlich der C₅-Aldosen wie Xylose, Ribose, Arabinose u.a., Desoxysacchariden wie Rhamnose, Fucose, Chinovose u.a., oder aminogruppenhaltigen Derivaten wie N-Acetyl-glucosamin, N-Acetyl-galactosamin, N-Acetyl-mannosamin u.a., sowie Oligosacchariden, insbesondere Disacchariden wie Lactose, Maltose, Cellobiose, Gentiobiose, u.a. oder auch höheren Oligosacchariden wie Maltotriose oder Maltotetraose, u.a. abgeleitete Verbindungen. Vorzugsweise werden peracylierte Saccharide eingesetzt, die als Anomerengemisch vorliegen können.

In einer besonders bevorzugten Ausführungsform des erfindungsgemäßen Verfahrens werden partiell acylierte Saccharide, die am anomeren Zentrum und einer weiteren Hydroxylgruppe verestert sind, und an den restlichen Hydroxylfunktionen resistente Schutzgruppen, beispielsweise (C₁-C₆)-äther- und/oder -estergruppen, tragen, in wasserfreiem Fluorwasserstoff, der das entsprechende Säureanhydrid enthält, fluoriert.

Als Acylreste kommen Acetyl-, Chloracetyl-, Trichloracetyl-, Bromacetyl-, Propionyl-, Butyryl-, Pivaloyl-, Benzoyl-, p-Methoxybenzoyl- und o- und p-Nitrobenzoylreste u.a. in Frage.

Bei dem erfindungsgemäßen Verfahren wird dem Fluorwasserstoff vor der Reaktion das entsprechende Carbonsäureanhydrid in einem Anteil von 2 bis 30 Vol.-%, bevorzugt 5 bis 20 Vol.-%, insbesondere 8 bis 12 Vol.-%, bezogen auf die Summe von Fluorwasserstoff und Carbonsäureanhydrid, zugefügt.

Das erfindungsgemäße Verfahren wird im allgemeinen bei einer Saccharid-Konzentration von 5 bis 80 Gew.-%, bevorzugt 20 bis 60 Gew.-%, insbesondere 40 bis 50 Gew.-%, durchgeführt.

Die Reaktionstemperatur liegt zweckmäßig zwischen etwa -20 bis etwa +20°C, bevorzugt bei -10 bis +10°C, besonders bevorzugt bei -2 bis +2°C.

Üblicherweise wird das erfindungsgemäße Verfahren bei Normaldruck ausgeführt. Es ist jedoch auch möglich bei Über- oder Unterdruck zu arbeiten.

Die Reaktionszeit des erfindungsgemäßen Verfahrens hängt naturgemäß von der Reaktivität der Ausgangsprodukte und der Temperatur ab, beträgt aber im allgemeinen höchstens 60 Minuten, vorzugsweise 30 Sekunden bis 40 Minuten, besonders bevorzugt 2 bis 5 Minuten.

Nach Erreichen des vollständigen Umsatzes, d.h. nach Ablauf der jeweiligen Reaktionszeit, wird das Reaktionsgemisch nach üblichen Methoden aufgearbeitet. Die Aufarbeitung erfolgt vorzugsweise umgehend nach Ablauf der Reaktionszeit. Es ist aber auch möglich, nach vollständigem Umsatz Weiterreaktionen durch Abkühlen der Lösung auf -50°C oder weniger zu unterdrücken und das Reaktionsgemisch zu einem späteren Zeitpunkt aufzuarbeiten.

Das erfindungsgemäße Verfahren wird durch die nachstehenden Beispiele erläutert, aber nicht eingeschränkt.

### Beispiele

### 1) Herstellung von 2,3,4,6-Tetra-O-acetyl-α-D-glucopyranosylfluorid

1 l (50 mol) wasserfreier Fluorwasserstoff wurde bei 0°C mit 100 ml (1,06 mol) Essigsäureanhydrid vorsichtig unter Rühren versetzt und anschließend 1100 g (2,82 mol) Glucosepentaacetat eingerührt. Nach einer Minute wurde die Reaktionslösung auf 5 kg Eis/Wasser gegossen, der ausgefallene Niederschlag abgesaugt, mit Natriumhydrogencarbonatlösung und Wasser ausgewaschen und im Vakuum getrocknet.
Ausbeute: 957 g (97 %) als weißes mikrokristallines Pulver
Reinheit: 98,2 % (GC)
Schmelzpunkt: 108°C (Lit.: F. Micheel, l.c. Schmp. 108°C)
Ohne Zusatz von Essigsäureanhydrid erhält man unter gleichen Bedingungen das Produkt in einer Ausbeute von 91 % und einer Reinheit von 90,6 %.

### 2) Herstellung von 2,3,4,6-Tetra-O-acetyl-α-D-galactopyranosylfluorid

100 ml (5 mol) wasserfreier Fluorwasserstoff wurde bei 0°C mit 10 ml (0,11 mol) Essigsäureanhydrid versetzt, und in die gekühlte Mischung 100 g (0,26 mol) Galactosepentaacetat gegeben. Nach beendeter Zugabe wurde die Reaktionslösung in ein kräftig gerührtes Gemisch aus 1 l Wasser, 500 g Eis und 300 ml Dichlormethan gegossen. Die organische Phase wurde abgetrennt, neutral gewaschen, getrocknet und eingeengt.
Ausbeute: 88,8 g (99 %) als farbloser Sirup
Reinheit 97,1 % (GC)
NMR (CDCl₃): 5,80 ppm (dd, H-1), J_{1,F} = 53,0, J_{1,2} = 2,7 Hz,
Ohne Zusatz von Essigsäureanhydrid erhält man unter gleichen Bedingungen das Produkt in einer Ausbeute von 98 % und einer Reinheit von 84 %.

### 3) 2,3,4,6-Tetra-O-acetyl-α-D-mannopyranosylfluorid

Entsprechend Beispiel 1 wurden 50 g (0,13 mol) Mannosepentaacetat mit 50 ml wasserfreier HF und 5 ml Essigsäureanhydrid umgesetzt.
Ausbeute: 41 g (91 %) als weißes, amorphes Pulver
Reinheit: 94,7 % (GC)
Ohne Zusatz von Essigsäureanhydrid erhält mag unter gleichen Bedingungen das Produkt in einer Ausbeute von 88 % und einer Reinheit von 85,0 %.

### 4) 2,3,4-Tri-O-acetyl-α-L-rhamnopyranosylfluorid

Entsprechend Beispiel 2 wurden 50 g (148 mmol) Tetraacetylrhamnose in 50 ml wasserfreier HF und 5 ml Essigsäureanhydrid umgesetzt.
Ausbeute: 42 g (97 %) als farbloser Sirup
Reinheit: 96,2 % (GC)
Ohne Zusatz von Essigsäureanhydrid erhält man unter gleichen Bedingungen das Produkt in einer Ausbeute von 89 % und einer Reinheit von 90,9 %.

### 5) 2,3,4-Tri-O-benzoyl-α-L-rhamnopyranosylfluorid

Zu 50 ml (2,5 mol) wasserfreier HF wurden bei 0°C 5 g (22 mmol) Benzoesäureanhydrid und anschließend 40 g (69 mmol) Tetrabenzoyl-L-rhamnose gegeben. Nach 20 min wurde die Lösung auf 200 g Eis mit 200 ml Wasser gegossen und von dem sich abscheidenden zähen Sirup dekantiert. Dieser Sirup wurde in Essigsäureäthylester gelöst, mit wäßriger Natriumhydrogencarbonat-Lösung gewaschen und mit basischem Ionentauscher von überschüssiger Benzoesäure befreit. Nach dem Eindampfen wurde ein farbloser Sirup, der beim Stehenlassen kristallisiert, erhalten.
Ausbeute: 29,9 g (91 %)
Reinheit: 94,3 % (GC)
Ohne Zusatz von Benzoesaureanhydrid erhält man unter gleichen Bedingungen das Produkt in einer Ausbeute von 88 % mit einer Reinheit von 86 %.

### 6) 2,3,6-Tri-O-acetyl-4-O-(2,3,4,6-tetra-O-acetyl-β-D-galactopyranosyl)-α-D-glucopyranosylfluorid-(Hepta-O-acetyl-α-D-lactosylfluorid)

Entsprechend Beispiel 2 wurden 100 ml wasserfreie HF mit 10 ml Essigsäureanhydrid gemischt und bei 0°C mit 50 g Lactoseoctaacetat umgesetzt. Die Reaktion war nach 5 min beendet.
Ausbeute: 45,6 g (97 %)
Reinheit: 94,2 % (GC)
Ohne Zusatz von Essigsäureanhydrid erhält man unter gleichen Bedingungen das Produkt in einer Ausbeute von 89 % mit einer Reinheit von 91,1 %.

### 7) 2,3,4-Tri-O-pivaloyl-α-D-xylopyranosylfluorid

Entsprechend Beispiel 2 wurden 20 ml wasserfreie HF mit 2 mol Pivalinsäureanhydrid gemischt und anschließend mit 10 g Tetrapivaloylxylose umgesetzt. Nach 30 min wurde die Lösung aufgearbeitet.
Ausbeute: 7,3 g (88 %)
Reinheit: 96,0 % (GC)

## Patentansprüche

1. Verfahren zur Herstellung von acylierten Glycosylfluoriden durch Fluorierung von Sacchariden, die wenigstens teilweise O-acyliert sind, in wasserfreiem Fluorwasserstoff, dadurch gekennzeichnet, daß dem Fluorwasserstoff vor der Reaktion das entsprechende Carbonsäureanhydrid zugesetzt und die Reaktionslösung nach Reaktionszeiten von höchstens 60 Minuten aufgearbeitet wird.

2. Verfahren nach Anspruch 1, dadurch gekennzeichnet, daß nach Reaktionszeiten von 30 Sekunden bis 40 Minuten, vorzugsweise von 2 bis 5 Minuten, aufgearbeitet wird.

3. Verfahren nach Anspruch 1 oder 2, dadurch gekennzeichnet, daß als partiell acylierte Saccharide solche, die am anomeren Zentrum und einer weiteren Hydroxylgruppe verestert sind, und an den restlichen Hydroxylfunktionen resistente Schutzgruppen tragen, eingesetzt werden.

4. Verfahren nach einem oder mehreren der Ansprüche 1 bis 3, dadurch gekennzeichnet, daß als peracylierte oder partiell acylierte Saccharide solche von Monosacchariden, Desoxysacchariden, aminogruppenhaltigen Derivaten oder Oligosacchariden abgeleitete Verbindungen eingesetzt werden.

5. Verfahren nach einem oder mehreren der Ansprüche 1 bis 4, dadurch gekennzeichnet, daß als Saccharide peracylierte Saccharide, eingesetzt werden.

6. Verfahren nach Anspruch 5, dadurch gekennzeichnet, daß die peracylierten Saccharide als Anomerengemisch vorliegen.

7. Verfahren nach einem oder mehreren der Ansprüche 1 bis 6, dadurch gekennzeichnet, daß es sich bei dem Acylrest um einen Acetylrest handelt.

8. Verfahren nach einem oder mehreren der Ansprüche 1 bis 7, dadurch gekennzeichnet, daß das Carbonsäureanhydrid in einem Anteil von 2 bis 30 Vol.-%, vorzugsweise 5 bis 20 Vol.-%, insbesondere 8 bis 12 Vol.-%, bezogen auf die Summe von Fluorwasserstoff und Carbonsäureanhydrid, zugesetzt wird.

9. Verfahren nach einem oder mehreren der Ansprüche 1 bis 8, dadurch gekennzeichnet, daß man das Saccharid in einer Konzentration von 5 bis 80 Gew.-%, vorzugsweise 20 bis 60 Gew.-%, insbesondere 40 bis 50 Gew.-%, einsetzt.

10. Verfahren nach einem oder mehreren der Ansprüche 1 bis 9, dadurch gekennzeichnet, daß die Reaktion bei einer Temperatur von -20 bis +20°C, vorzugsweise bei -10 bis +10°C, besonders bevorzugt bei -2 bis +2°C, durchgeführt wird.

11. Verfahren nach einem oder mehreren der Ansprüche 1 bis 10, dadurch gekennzeichnet, daß die Reaktion bei Normaldruck durchgeführt wird.

## Claims

1. Process for preparing acylated glycosyl fluorides by fluorination of saccharides, which are at least partially O-acylated, in anhydrous hydrogen fluoride, characterized in that the corresponding carboxylic anhydride is added to the hydrogen fluoride before the reaction and the reaction solution is processed after reaction times of at most 60 minutes.

2. Process according to Claim 1, characterized in that processing occurs after reaction times of 30 seconds to 40 minutes, preferably of 2 to 5 minutes.

3. Process according to Claim 1 or 2, characterized in that the partially acylated saccharides employed are those which are esterified at the anomeric center and at one further hydroxyl group and which carry resistant protective groups on the remaining hydroxyl functions.

4. Process according to one or more of Claims 1 to 3, characterized in that the peracylated or partially acylated saccharides employed are those which are compounds derived from monosaccharides, deoxysaccharides, derivatives that contain amino groups, or oligosaccharides.

5. Process according to one or more of Claims 1 to 4, characterized in that peracylated saccharides are employed as saccharides.

6. Process according to Claim 5, characterized in that the peracylated saccharides are present as an anomeric mixture.

7. Process according to one or more of Claims 1 to 6, characterized in that the acyl radical is an acetyl radical.

8. Process according to one or more of Claims 1 to 7, characterized in that the carboxylic anhydride is added in a proportion of 2 to 30 vol.%, preferably 5 to 20 vol.%, in particular 8 to 12 vol.%, based on the sum of hydrogen fluoride and carboxylic anhydride.

9. Process according to one or more of Claims 1 to 8, characterized in that the saccharide is employed in a concentration of 5 to 80 % by weight, preferably 20 to 60 % by weight, in particular 40 to 50 % by weight.

10. Process according to one or more of Claims 1 to 9, characterized in that the reaction is carried out at a temperature of -20 to +20°C, preferably at -10 to +10°C, particularly preferably at -2 to +2°C.

11. Process according to one or more of Claims 1 to 10, characterized in that the reaction is carried out at atmospheric pressure.

## Revendications

1. Procédé pour préparer des fluorures de glycosyle acylés par fluoration dans de l'acide fluorhydrique anhydre de saccharides qui sont au moins partiellement O-acylés, le procédé étant caractérisé en ce que l'on ajoute à l'acide fluorhydrique, avant la réaction, l'anhydride de l'acide carboxylique correspondant et en ce que l'on traite la solution de réaction après des temps de réaction d'au plus 60 minutes.

2. Procédé selon la revendication 1, caractérisé en ce que l'on effectue le traitement après des temps de réaction de 30 secondes à 40 minutes, de préférence de 2 à 5 minutes.

3. Procédé selon la revendication 1 ou 2, caractérisé en ce que l'on utilise comme saccharides partiellement acylés ceux qui sont estérifiés sur le centre anomère et sur un autre coupe hydroxyle, et qui portent des groupes de protection résistants sur les fonctions hydroxyle restantes.

4. Procédé selon une ou plusieurs des revendications 1 à 3, caractérisé en ce que, comme saccharides peracylés ou partiellement acylés, on utilise ceux des monosaccharides, des désoxysaccharides, des dérivés comportant des groupes amino ou des composés dérivés des oligosaccharides.

5. Procédé selon une ou plusieurs des revendications 1 à 4, caractérisé en ce que l'on utilise comme saccharides les saccharides peracylés.

6. Procédé selon la revendication 5, caractérisé en ce que les saccharides peracylés se présentent comme un mélange d'anomères.

7. Procédé selon une ou plusieurs des revendications 1 à 6, caractérisé en ce que, pour le radical acyle, il s'agit d'un radical acétyle.

8. Procédé selon une ou plusieurs des revendications 1 à 7, caractérisé en ce que l'on ajoute l'anhydride de l'acide carboxylique dans une quantité de 2 à 30 % en volume, de préférence de 5 à 20 % en volume, en particulier de 8 à 12 % en volume, calculée sur la somme de l'acide fluorhydrique et de l'anhydride carboxylique.

9. Procédé selon une ou plusieurs des revendications 1 à 8, caractérisé en ce que l'on utilise le saccharide dans une concentration de 5 à 80 % en poids, de préférence de 20 à 60 % en poids, en particulier de 40 à 50 % en poids.

10. Procédé selon une ou plusieurs des revendications 1 à 9, caractérisé en ce que l'on met en oeuvre la réaction à une température de -20 à +20°C, de préférence de -10 à +10°C, de façon particulièrement préférée de -2 à +2°C.

11. Procédé selon une ou plusieurs des revendications 1 à 10, caractérisé en ce que la réaction est mise en oeuvre à la pression normale.
